(19) Europäisches Patentamt
European Patent Office
Office européen des brevets

(11) Publication number : **0 328 862 B1**

(12) # EUROPEAN PATENT SPECIFICATION

(45) Date of publication of patent specification : **13.05.92 Bulletin 92/20**

(21) Application number : **89100305.5**

(22) Date of filing : **10.01.89**

(51) Int. Cl.$^5$ : **A61K 47/00,** A61K 9/00, A61K 31/535, C09K 9/02, // A61K47/02, C07D498/18 , (C07D498/18, 265:00, 265:00, 203:00)

(54) **A stabilizing agent and an injectable composition containing the same.**

(30) Priority : **12.01.88 JP 5423/88**

(43) Date of publication of application : **23.08.89 Bulletin 89/34**

(45) Publication of the grant of the patent : **13.05.92 Bulletin 92/20**

(84) Designated Contracting States : **AT BE CH DE ES FR GB GR IT LI LU NL SE**

(56) References cited :
**EP-A- 0 123 291**
**EP-A- 0 166 389**
**CHEMICAL ABSTRACTS, vol.101,no.8, 20th August 1984, page 299, abstract no. 60042d; V.DAS GUPTA et al.: "Stability ofdobutamine hydrochloride and veranpamil hydrochloride in 0,9 % sodium chloride and 5% dextrose injections"; &AM.J.HOSP.PHARM.41(4), 686-689**
**PATENT ABSTRACTS OF JAPAN, vol. 10, no. 156, 5th June 1986 (C-351) (2212); &JP-A-61 10590 (FUJISAWA YAKUHIN KOGYO K.K.)**

(73) Proprietor : **FUJISAWA PHARMACEUTICAL CO., LTD.**
**3, Doshomachi 4-chome Higashi-ku**
**Osaka-shi Osaka 541 (JP)**

(72) Inventor : **Mochizuki, Yoshiaki**
**1-1-7-706, Aikawa, Higashiyodogawa-ku**
**Osaka-shi Osaka 533 (JP)**
Inventor : **Imasaki, Takshi**
**2-2-10, Midorigaoka**
**Ideka-shi Osaka 563 (JP)**
Inventor : **Nasu, Hiroshi**
**1-12, Niitaka Yodogawa-ku**
**Osaka-shi Osaka 532 (JP)**

(74) Representative : **Türk, Gille, Hrabal**
**Brucknerstrasse 20**
**W-4000 Düsseldorf 13 (DE)**

EP 0 328 862 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a pharmaceutical composition comprising sodium chloride and a tetracyclo compound which is of value as a drug.

More particularly, this invention relates, in one aspect, to a pharmaceutical composition comprising sodium chloride and a tetracyclo compound of the formula:

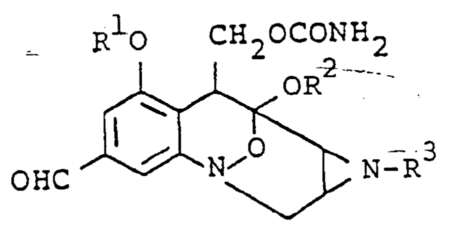

(I)

wherein $R^1$, $R^2$ and $R^3$ each means a ($C_1$-$C_6$) alkanoyl group and, in another aspect, to an injectable composition comprising said tetracyclo compound (I) and sodium chloride.

In the manufacture of injectable pharmaceutical preparations, it is a well-known practice to add sodium chloride for isotonization purposes but the addition of sodium chloride for improving the storage stability and/or re-solubility of the active ingredient is new to the industry.

Since the administration of a drug by injection insures the systemic effect of the drug without involving the gastrointestinal tract, this method of administration is very frequently relied on in clinical practice as a method for assuring the most positive therapeutic efficacy against various diseases.

However, since it is not rare that a fairly long interval is involved between the manufacture of a drug and the administration of it to a patient, the efficacy and safety of the drug must be impeccably assured notwithstanding a prolonged storage period. The efficacy and safety of a drug depends in a large measure on its stability and in order that a pharmaceutical preparation may be a useful drug product, its stability is an important factor.

The tetracyclo compound (I) as an active ingredient of the injectable composition of this invention is a very useful drug as such but has the drawback in terms of stability that it is unstable in solution on the one hand and, when lyophilized and stored, tends to be degraded and become poor in re-solubility on the other hand.

The inventors of this invention found, after intensive and diligent research, that sodium chloride not only improves the stability of tetracyclo compound (I) remarkably but also improves the re-solubility of the drug and implemented the findings into this invention.

It is, therefore, and object of this invention to provide a pharmaceutical composition comprising sodium chloride and tetracyclo compound (I).

It is another object of this invention to provide an injectable composition characterized by comprising said tetracyclo compound (I) and, as a stabilizing agent therefor, sodium chloride.

The tetracyclo compound (I) used as an active ingredient in this invention is of value as a drug, for example as an antitumor agent or an antimicrobial agent, and is a known compound together with the process for production thereof, as described in Japanese Patent Unexamined Publication No. 10590/1986, for instance. Particularly the compound (I) wherein $R^1$, $R^2$ and $R^3$ are acetyl groups has been named FR-66973 Substance and is known to have high antitumor activity.

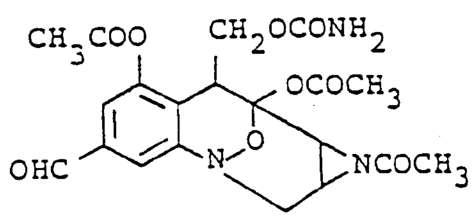

(FR-66973 Substance)

Suitable examples of the lower alkanoyl group for $R^1$, $R^2$ and $R^3$ in tetracyclo compound (I) include, in addition to acetyl mentioned above, other aliphatic acyl groups of 1 to 6 carbon atoms such as formyl, propionyl, butyryl, isobutyryl, valeryl, isovaleryl, pivaloyl, hexanoyl and so on. Preferred are $C_1$-$C_4$ alkanoyl groups, acetyl being the most desirable group.

While the tetracyclo compound (I) may include one or more stereoisomers such as optical isomers due to the asymmetric carbon atom(s), such isomers are also subsumed in the context of this invention.

For the purposes of this invention, sodium chloride may be of any grade as long as it can be incorporated in injectable composition.

In the injectable composition of this invention, there may be incorporated, in addition to sodium chloride, other stabilizers, isotonizing agents such as mannitol and xylitol and, if necessary, even other additives such as pH adjusting agents or buffers.

The pharmaceutical composition and the injectable composition of this invention can be manufactured by the established pharmaceutical procedure which is capable of mixing said tetracyclo compound (I) and sodium chloride homogenously. For example, such a composition can be obtained by dissolving tetracyclo compound (I) in water, an organic solvent such as acetone, dioxane, acetonitrile, ethanol, isopropyl alcohol, or a mixture thereof, then adding the stabilizing agent sodium chloride, and if necessary adding an appropriate amount of water thereto to provide a homogenous solution and lyophilizing the resulting solution.

In the injectable composition of this invention, the relative amount of tetracyclo compound (I) and sodium chloride is not critical but the proportion of sodium chloride should be sufficient to improve the stability of tetracyclo compound (I). However, to prepare an isotonic solution in a total volume suited to administration, the weight ratio of tetracyclo compound to sodium chloride is preferably 1:2 through 1:50 and more desirably 1:5 through 1:30.

By virtue of the stabilizing agent of this invention, the storage stability of the active ingredient tetracyclo compound (I) is remarkably improved and, at the same time, the re-solubility of the same after storage is also markedly improved.

The following,examples are merely illustrative and by no means limitative of this invention.

Example 1

| | |
|---|---|
| FR-66973 Substance | 5 mg |
| Sodium chloride JP | 45 mg |
| Total | 50 mg |

(JP = Japanese Pharmacopeia)

FR-66973 Substance (5 mg) and sodium chloride JP (45 mg) were added to acetone (0.25 ml) followed by addition of distilled water for injection to make 1 ml. After dissolution at 20°C, the solution was aseptically filtered through a Teflon filter. The filtrate was filled into a vial and lyophilized to give an injectable composition containing the above ingredients.

Example 2

| | |
|---|---|
| FR-66973 Substance | 5 mg |
| Sodium chloride JP | 90 mg |
| Total | 95 mg |

In the same manner as Example 1, an injectable composition containing the above-mentioned ingredients was obtained.

To prepare an injection from each of the injectable composition obtained in Examples 1 and 2, a suitable amount of distilled water for injection can be added to the injectable composition to make it isotonic to the blood and the resulting isotonic solution can be administered.

Test Examples

To prove the storage stability-improving effect of sodium chloride on tetracyclo compound (I), the following storage stability test was performed.

[A] Storage stability test

Using the injectable compositions obtained in Examples 1 and 2 as test materials, and for purposes of comparison, a lyophilized product containing FR-66973 Substance (5 mg) only was used as a control material. Each material was stored under the various conditions indicated in Table 1 and the residual amount of FR-66973 Substance was determined by the following method.

[Method of determination]

Each sample was dissolved using a 28% aqueous solution of acetonitrile (total volume 50 ml) and FR-66973 Substance was assayed by liquid chromatography on octadecylsilylized silicone-coated silica gel using a perchlorate buffer containing 28% acetonitrile as an eluent. The results are shown in Table 1.

Table 1. The storage stability improving effect of sodium chloride on FR-66973 Substance

| Storage conditions | | Residual amount (%) of FR-66973 Substance | | |
|---|---|---|---|---|
| Temperature | Period | Control | Example 1 | Example 2 |
| 80°C | 4 Days | 80.6 | 94.3 | 102.0 |
| | 7 Days | 62.9 | 84.2 | 94.5 |
| 60°C | 20 Days | 72.4 | 97.7 | 97.7 |
| | 1 Month | 70.5 | 89.0 | 90.4 |
| | 2 Months | 57.4 | 86.6 | 88.4 |

[B] Re-solubility test

The test was performed using the injectable composition of Example 1 as a test material and a lyophilizate containing FR-66973 Substance (5 mg) alone as a control material. Each material was stored under the various conditions shown in Table 2 and, then, distilled water for injection (5 ml) was added. The mixture was shaken and the state of dissolution was examined with the naked eye after a given time period.

For further confirmation of solubilizing effect, the re-solubility of the injectable composition of Example 1 and that of the control immediately after preparation was also investigated. The results are shown in Table 2.

Table 2. The re-solubility improving effect of sodium chloride on FR-66973 Substance

| Storage conditions | | Re-solubility | |
|---|---|---|---|
| Temperature | Period | Control | Example 1 |
| 80°C | 4 Days | x | o |
| | 7 Days | x | o |
| | 20 Days | x | o |
| 60°C | 20 Days | x | o |
| | 1 Month | x | o |
| 40°C | 1 Month | o | o |
| | 3 Months | ▵ | o |
| | 6 Months | x | o |
| Immediately after preparation of the sample | | o | o |

o---rapidly dissolved (15-30 seconds)

▵---dissolved but dissolution fairly retarded (2-5 minutes)

x---not dissolved even by shaking for 30 minutes

It is apparent from the above results of the storage stability and re-solubility tests that the addition of sodium chloride markedly improves the storage stability and re-solubility of tetracyclo compound (I) and that an injectable composition with improved storage stability and re-solubility can be obtained by adding sodium chloride as a stabilizing agent.

**Revendications**

**Claims for the following Contracting States: AT, BE, CH, DE, FR, GB, IT, LI, LU, NL, SE**

1. A pharmaceutical composition comprising sodium chloride and a tetracyclo compound of the formula:

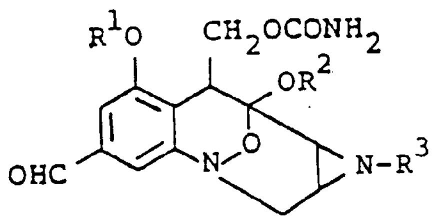

wherein $R^1$, $R^2$ and $R^3$ each means a $C_1$-$C_6$ alkanoyl group.

2. The pharmaceutical composition claimed in Claim 1, wherein $R^1$, $R^2$ and $R^3$ each is an acetyl group.

3. An injectable composition comprising a tetracyclo compound of the formula:

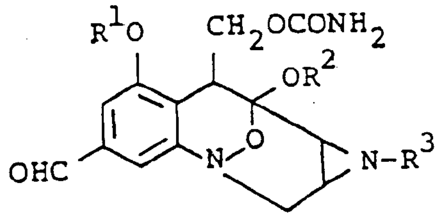

wherein $R^1$, $R^2$ and $R^3$ each means a $C_1$-$C_6$ alkanoyl group, and sodium chloride.

4. The injectable composition claimed in Claim 3, wherein the weight ration of tetracyclo compound to sodium chloride is 1:2 to 1:50.

5. The injectable composition claimed in Claim 4, wherein $R^1$, $R^2$ and $R^3$ each is an acetyl group.

6. A process for producing the pharmaceutical composition of claim 1, which comprises mixing a tetracyclo compound of the formula:

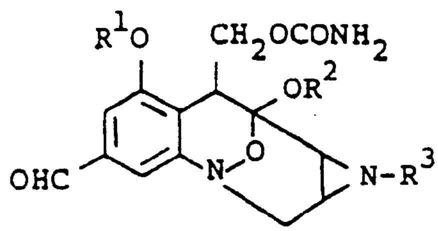

wherein $R^1$, $R^2$ and $R^3$ each means a $C_1$-$C_6$ alkanoyl group, and sodium chloride homogenously.

7. The process for producing an pharmaceutical composition claimed in Claim 6, wherein the weight ratio of tetracyclo compound to sodium chloride is 1:2 to 1:50.

8. The process for producing an pharmaceutical composition claimed in Claim 7, which comprises dissolving said tetracyclo compound and sodium chloride in a solvent, and lyophilizing the resulting solution.

9. The process for producing an pharmaceutical composition claimed in Claim 8, wherein $R^1$, $R^2$ and $R^3$ each is an acetyl group.

10. A method for improving the storage stability and re-solubility of a tetracyclo compound of the formula:

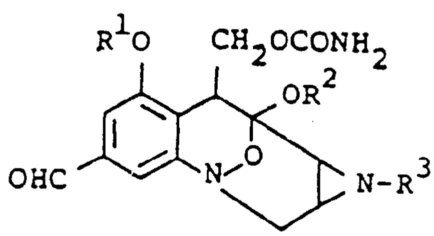

wherein $R^1$, $R^2$ and $R^3$ each means a $C_1$-$C_6$ alkanoyl group, which comprises mixing said tetracyclo compound and sodium chloride homogenously.

11. The method claimed in Claim 10, wherein the weight ratio of tetracyclo compound to sodium chloride is 1:2 through 1:50.

12. The method claimed in Claim 11, which comprises dissolving said tetracyclo compound and sodium chloride in a solvent, and lyophilizing the resulting solution.

13. The method claimed in Claim 12, wherein $R^1$, $R^2$ and $R^3$ each is an acetyl group.

**Claims for the following Contracting States: ES, GR**

1. A process for producing a pharmaceutical composition which comprises mixing a tetracyclo compound of the formula:

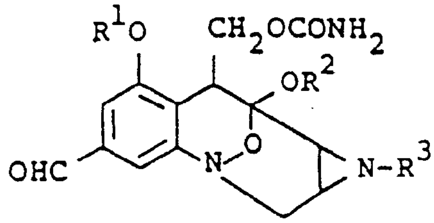

wherein $R^1$, $R^2$ and $R^3$ each means a $C_1$-$C_6$ alkanoyl group, and sodium chloride homogenously.

2. The process for producing a pharmaceutical composition claimed in Claim 1, wherein the weight ratio of tetracyclo compound to sodium chloride is 1:2 to 1:50.

3. The process for producing a pharmaceutical composition claimed in Claim 2, which comprises dissolving said tetracyclo compound and sodium chloride in a solvent, and lyophilizing the resulting solution.

4. The process for producing a pharmaceutical composition claimed in Claim 3, wherein $R^1$, $R^2$ and $R^3$ each is an acetyl group.

## Patentansprüche

## Patentansprüche für folgenden Vertragsstaaten: AT, BE, CH, DE, FR, GB, IT, LI, LU, NL, SE

1. Pharmazeutische Zusammensetzung, umfassend Natriumchlorid und eine Tetracycloverbindung der Formel:

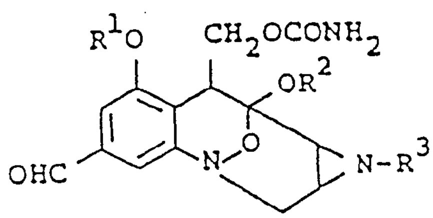

worin $R^1$, $R^2$ und $R^3$ jeweils eine $C_1$-$C_6$-Alkanoylgruppe darstellen.

2. Pharmazeutische Zusammensetzung nach Anspruch 1, worin $R^1$, $R^2$ und $R^3$ jeweils eine Acetylgruppe darstellt.

3. Injizierbare Zusammensetzung, umfassend eine Tetracycloverbindung der Formel:

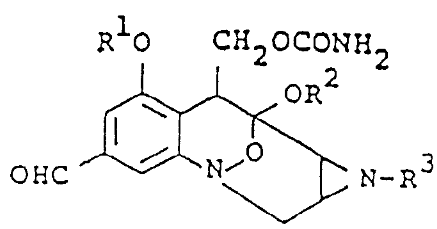

worin $R^1$, $R^2$ und $R^3$ jeweils eine $C_1$-$C_6$-Alkanoylgruppe darstellen, und Natriumchlorid.

4. Injizierbare Zusammensetzung nach Anspruch 3, worin das Gewichtsverhältnis von Tetracycloverbindung zu Natriumchlorid 1:2 bis 1:50 beträgt.

5. Injizierbare Zusammensetzung nach Anspruch 4, worin $R^1$, $R^2$ und $R^3$ jeweils eine Acetylgruppe sind.

6. Verfahren zur Herstellung der pharmazeutischen Zusammensetzung von Anspruch 1, gekennzeichnet durch homogenes Vermischen einer Tetracycloverbindung der Formel

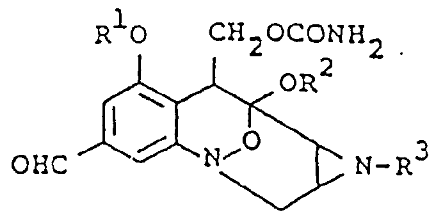

worin $R^1$, $R^2$ und $R^3$ jeweils eine $C_1$-$C_6$-Alkanoylgruppe darstellen, und Natriumchlorid.

7. Verfahren zur Herstellung einer pharmazeutischen Zusammensetzung nach Anspruch 6, worin das Gewichtsverhältnis von Tetracycloverbindung zu Natriumchlorid 1:2 bis 1:50 beträgt.

8. Verfahren zur Herstellung einer pharmazeutischen Zusammensetzung nach Anspruch 7, gekennzeichnet durch Lösen der Tetracycloverbindung und des Natriumchlorids in einem Lösungsmittel und Lyophilisieren der erhaltenen Lösung.

9. Verfahren zur Herstellung einer pharmazeutischen Zusammensetzung nach Anspruch 8, worin $R^1$, $R^2$ und $R^3$ jeweils eine Acetylgruppe darstellen.

10. Verfahren zur Verbesserung der Lagerungsstabilität und der Lösbarkeit einer Tetracycloverbindung der Formel

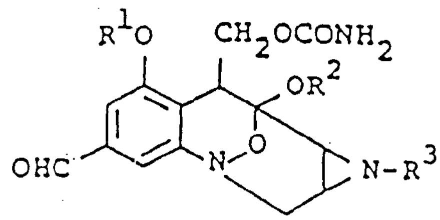

worin $R^1$, $R^2$ und $R^3$ jeweils eine $C_1$-$C_6$-Alkanoylgruppe bedeuten, gekennzeichnet durch homogenes Vermischen der Tetracycloverbindung und des Natriumchlorids.

11. Verfahren nach Anspruch 10, worin das Masseverhältnis von Tetracycloverbindung zu Natriumchlorid 1:2 bis 1:50 beträgt.

12. Verfahren nach Anspruch 11, gekennzeichnet durch Lösen der Tetracycloverbindung und des Natriumchlorids in einem Lösungsmittel und Lyophilisieren der erhaltenen Lösung.

13. Verfahren nach Anspruch 12, worin $R^1$, $R^2$ und $R^3$ jeweils eine Acetylgruppe darstellen.

**Patentansprüche für folgenden Vertragsstaaten: ES, GR**

1. Verfahren zur Herstellung einer pharmazeutischen Zusammensetzung, gekennzeichnet durch homogenes Vermischen einer Tetracycloverbindung der Formel

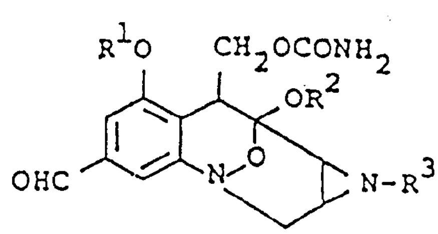

worin $R^1$, $R^2$ und $R^3$ jeweils eine $C_1$-$C_6$-Alkanoylgruppe darstellen, und Natriumchlorid.

2. Verfahren zur Herstellung einer pharmazeutischen Zusammensetzung nach Anspruch 1, worin das Gewichtsverhältnis von Tetracycloverbindung zu Natriumchlorid 1:2 bis 1:50 beträgt.

3. Verfahren zur Herstellung einer pharmazeutischen Zusammensetzung nach Anspruch 2, gekennzeichnet durch Lösen der Tetracycloverbindung und des Natriumchlorids in einem Lösungsmittel und Lyophilisieren der erhaltenen Lösung.

4. Verfahren zur Herstellung einer injizierbaren pharmazeutischen Zusammensetzung nach Anspruch 3, worin $R^1$, $R^2$ und $R^3$ jeweils eine Acetylgruppe darstellen.

## Revendications

### Revendications pour les Etats contractants suivants: AT, BE, CH, DE, FR, GB, IT, LI, LU, NL, SE

1. Composition pharmaceutique comprenant du chlorure de sodium et un composé tétracyclo de la formule :

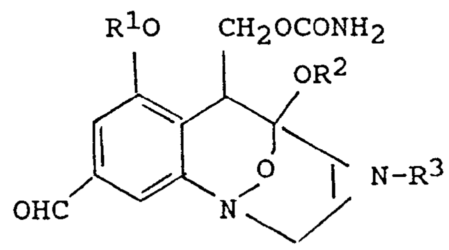

où $R^1$, $R^2$ et $R^3$ représentent chacun un groupe alcanoyle $C_1$-$C_6$.

2. Composition pharmaceutique revendiquée dans la revendication 1, dans laquelle $R^1$, $R^2$ et $R^3$ sont chacun un groupe acétyle.

3. Composition injectable comprenant un composé tétracyclo de la formule :

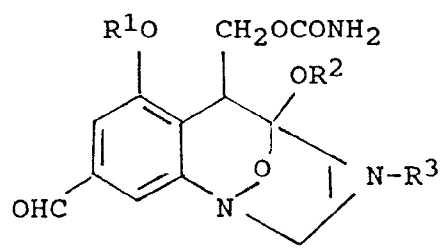

où $R^1$, $R^2$ et $R^3$ représentent chacun un groupe alcanoyle $C_1$-$C_6$ et du chlorure de sodium.

4. Composition injectable revendiquée dans la revendication 3, dans laquelle le rapport pondéral du composé tétracyclo et du chlorure de sodium est de 1:2 à 1:50.

5. Composition injectable revendiquée dans la revendication 4, dans laquelle $R^1$, $R^2$ et $R^3$ sont chacun un groupe acétyle.

6. Procédé pour produire la composition pharmaceutique de la revendication 1, qui comprend de mélanger d'une manière homogène un composé tétracyclo de la formule :

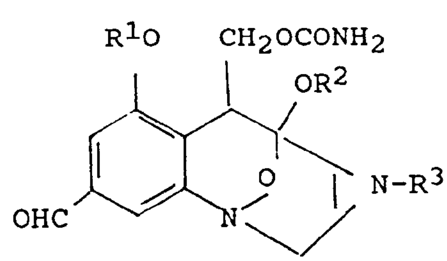

où $R^1$, $R^2$ et $R^3$ représentent chacun un groupe alcanoyle $C_1$-$C_6$, et du chlorure de sodium.

7. Procédé pour produire une composition pharmaceutique revendiqué dans la revendication 6, dans lequel le rapport pondéral du composé tétracyclo et du chlorure de sodium est de 1:2 à 1:50.

8. Procédé pour produire une composition pharmaceutique revendiqué dans la revendication 7, qui comprend de dissoudre ledit composé tétracyclo et du chlorure de sodium dans un solvant et de lyophiliser la solution résultante.

9. Procédé pour produire une composition pharmaceutique revendiqué dans la revendication 8, dans lequel

$R^1$, $R^2$ et $R^3$ sont chacun un groupe acétyle.

10. Méthode pour améliorer la stabilité en cours de conservation et la redissolution d'un composé tétracyclo de la formule :

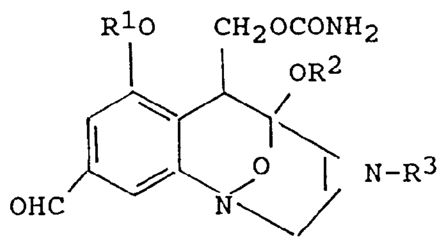

où $R^1$, $R^2$ et $R^3$ représentent chacun un groupe alcanoyle $C_1$-$C_6$, qui comprend de mélanger d'une manière homogène ledit composé tétracyclo et du chlorure de sodium.

11. Méthode revendiquée dans la revendication 10, dans laquelle le rapport pondéral de composé tétracyclo et de chlorure de sodium est de 1:2 à 1:50.

12. Méthode revendiquée dans la revendication 11, qui comprend de dissoudre ledit composé tétracyclo et du chlorure de sodium dans un solvant et de lyophiliser la solution résultante.

13. Méthode revendiquée dans la revendication 12, dans laquelle $R^1$, $R^2$ et $R^3$ sont chacun un groupe acétyle.

**Revendications pour les Etats contractants suivants: ES, GR**

1. Procédé pour produire une composition pharmaceutique qui comprend de mélanger d'une manière homogène un composé tétracyclo de la formule :

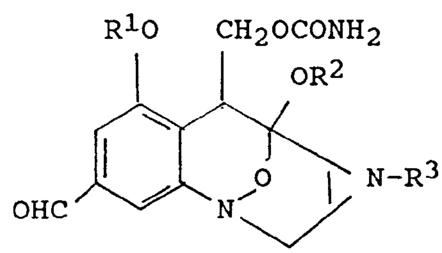

où $R^1$, $R^2$ et $R^3$ représentent chacun un groupe alcanoyle $C_1$-$C_6$, et du chlorure de sodium.

2. Procédé pour produire une composition pharmaceutique revendiqué dans la revendication 1, dans lequel le rapport pondéral du composé tétracyclo et du chlorure de sodium est de 1:2 à 1:50.

3. Procédé pour produire une composition pharmaceutique revendiqué dans la revendication 2, qui comprend de dissoudre ledit composé tétracyclo et du chlorure de sodium dans un solvant et de lyophiliser la solution résultante.

4. Procédé pour produire une composition pharmaceutique revendiqué dans la revendication 3, dans lequel $R^1$, $R^2$ et $R^3$ sont chacun un groupe acétyle.